**Europäisches Patentamt**

**European Patent Office**

**Office européen des brevets**

(19)

(11) Veröffentlichungsnummer : **0 069 048**
**B1**

(12)

# EUROPÄISCHE PATENTSCHRIFT

(45) Veröffentlichungstag der Patentschrift :
06.05.87

(51) Int. Cl.⁴ : **A 61 B 17/58**

(21) Anmeldenummer : **82730083.1**

(22) Anmeldetag : **18.06.82**

(54) **Nagel zum Fixieren einer Oberschenkelfraktur.**

(30) Priorität : **18.06.81 DE 3124059**
**09.07.81 DE 3127378**

(43) Veröffentlichungstag der Anmeldung :
**05.01.83 Patentblatt 83/01**

(45) Bekanntmachung des Hinweises auf die Patenterteilung : **06.05.87 Patentblatt 87/19**

(84) Benannte Vertragsstaaten :
**AT BE CH DE FR GB IT LI NL SE**

(56) Entgegenhaltungen :
**DE-A- 874 637**
**DE-A- 2 527 460**
**US-A- 4 055 172**
**BIOMEDIZINISCHE TECHNIK, Band 26, Nr. 11,**
**November 1981, Seiten 283-285, Berlin, DE. C.**
**KRANZ: "Vergleichende experimentelle Untersu-**
**chung von neu entwickelten Condylennagelenden"**

(73) Patentinhaber : **MECRON MEDIZINISCHE PRODUKTE**
**GMBH**
**Nunsdorfer Ring 25-27**
**D-1000 Berlin 48 (DE)**

(72) Erfinder : **Anapliotis, Emanuel**
**Hauptstrasse 76**
**D-1000 Berlin 41 (DE)**

(74) Vertreter : **Christiansen, Henning, Dipl.-Ing.**
**Dietrich-Schäfer-Weg 21**
**D-1000 Berlin 41 (DE)**

## Beschreibung

Die Erfindung betrifft einen Knochennagel der im Oberbegriff des Anspruchs 1 angegebenen Art, ein Werkzeug zum Ausüben einer Kraft bzw. eines Drehmoments auf einen derartigen Nagel.

Das Prinzip der « Bündel-Nagelung » ist beschrieben in der Monographie « Die Bündel-Nagelung », von K.H. Hackethal, Berlin, Göttingen, Heidelberg 1961. Dabei wird eine stabile, formgerechte Markraumschienung durch eine Anzahl elastischer Stahlnägel erzielt, welche durch eine Öffnung des Markraums eingeschlagen wird. Das Bündel der — meist gebogenen — Nägel füllt den Markraum ganz oder teilweise aus und bewirkt durch eine Spreizung der Nägel im Bereich des proximalen Bündelendes eine Stabilisierung und damit eine Erhaltung des Repositionserfolges (aaO., Seite 25, Abb. 13 und zugehöriger Text). Bei der Bündelnagelung wird die Federwirkung der Nägel ausgenutzt, welche sich u. a. an der Kortikalis im Bereich des zum Einführen der Nägel geschaffenen Knochenfensters abstützen. Die Erhaltung der Vorspannung der Nägel nach deren Positionierung ist eine wichtige Voraussetzung für den Therapieerfolg.

Alle Manipulationen der Nägel beim Einschlagen, Positionieren und Herausziehen erfolgen von ihrem distalen Ende her. Bei der Handhabung müssen infolgedessen folgende Bewegungen durch entsprechende Krafteinwirkungen übertragen werden :

1. Einschlagkräfte, welche stoßartig vom rückwärtigen Ende des Nagels in dessen Längsrichtung wirken,

2. Drehmomentkräfte, welche beim Einschlagen aufgebracht werden und eine Verdrehung des Nagels beim Eintreiben ermöglichen,

3. Rückzugskräfte, welche ein Herausziehen des Nagels aus dem Markraum nach der Knochenheilung ermöglichen.

Zur Übertragung dieser Kräfte sind entweder Spezialwerkzeuge erforderlich, welche konstruktiv aufwendig ausgebildet sein müssen (aaO., Seite 95, Abb. 66 und 67), und bei ihrer Benutzung Raum benötigen, der wegen der gebündelten Anordnung der Nägel nur im beschränkten Maße zur Verfügung steht.

Aus der deutschen Auslegeschrift 24 59 257 ist es bekannt, Knochennägel an ihrem distalen Ende plättenförmig abgeflacht auszubilden, so daß sich für mehrere Nagelenden neben- und übereinander angeordnet sein können. Durch die « plättchenförmige Abflachung » ist die Möglichkeit der Übertragung eines Drehmoments beim Einschlagen des Nagels mittels eines Vorschlageisens gegeben. Diese « plättchenförmige Abflachung » soll darüberhinaus dafür sorgen, daß die aufgrund der Vorspannung des Nagels an seinem distalen Ende in den Knochen einzuleitenden Kräfte über eine vergrößerte Fläche auf den Knochen übertragen werden.

Weiterhin ist es aus dem deutschen Gebrauchsmuster 75 19 604 ein Nagel mit den im Oberbegriff des Anspruchs 1 angegebenen Merkmalen bekannt.

Dabei ist jedoch nicht berücksichtigt, daß es unter Operationsbedingungen nicht in allen Fällen möglich ist, die distalen Enden der Nägel so zu positionieren, daß sie an der Kortikalis glatt anliegen und die Federspannung des Nagels über die gesamte Fläche der Abflachung in den Knochen eingeleitet wird. Eine derartige optimale Positionierung ist schon deshalb vom Grundsatz her ausgeschlossen, weil dem Verfahren der Bündelnagelung das Prinzip zugrundeliegt, daß die Nagelspitzen sich räumlich fächerförmig an der Spongiosa des Femur-Kopfes aufspreizen, um eine korrekte Repositionierung und Stabilität der Fraktur zu erreichen. Da die bekannten Nägel mit plättchenförmiger Abflachung in Nagellängsrichtung eine Krümmung aufweisen, die in Bezug auf die Abflachung bei allen Nägeln gleichgerichtet ist, ergeben sich zwangsläufig für die plättchenförmigen Abflachungen Positionen, die von einer parallelen, dachziegelartigen Anordnung abweichen. Damit besteht die Gefahr, daß die Nägel mit der Schmalseite der Abflachung, d.h. mit einer scharfen Kante meisselartig Druck auf die Kortikalis ausüben, wodurch die Gefahr einer Perforation gegeben ist.

Da die distalen Enden der Nägel im fixierten Zustand aus der erzeugten Fensteröffnung im Knochen hervorstehen und den Rand des Fensters als « Rückenlehne » benutzen, so daß ein Zurückfedern in den Markraum hinein verhindert ist, besteht außerdem die Gefahr, daß bei nicht optimal ausgerichteten abgeflachten Nagelenden diese im Bereich ihrer scharfen Kanten einen Hautdekubitus mit möglicher späterer Infektion hervorrufen.

Der im Anspruch 1 angegebenen Erfindung liegt die Aufgabe zugrunde, eine Gestaltung für das distale Ende der Knochennägel entsprechend dem Oberbegriff anzugeben, bei der eine derartige Gefahr nicht gegeben ist, welche aber trotzdem die Aufnahme aller bei der Manipulation des Nagels zu übertragenden Kräfte erlaubt, so daß insbesondere beim osteoporotischen Knochen ein Einbrechen des Nagels in den Knochen im Bereich der Kortikalis verhindert ist.

Die Erfindung beruht dabei auf der Erkenntnis, daß eine meißelartige Wirkung auf den umgebenden Knochenbereich bzw. umgebende Gewebebereiche nur dann verhindert ist, wenn scharfkantige Abwinkelungen und hohe Anpreßdrücke vermieden sind. Der gefundene Lösungweg weicht dabei ab von dem Grundsatz, daß insbesondere zur Drehmomentübertragung mittels schraubenschlüsselartiger Werkzeuge in der Technik üblicherweise scharfkantige Profile (3-, 4- oder Mehrkant-) gewählt werden. Es mußte also eine Formgestaltung gefunden werden, welche eine dichte Bündelpackung erlaubt, ohne daß meißelartige, schmale Flächenkanten entstehen.

Da die Ausnehmung an derjenigen Seite angeordnet ist, welche sich entgegengesetzt zu derjenigen befindet, die aufgrund der Biegung des Nagels dazu bestimmt ist, bei eingetriebenem Nagel an der Kortikalis in Rückenlage anzuliegen, wird eine Knochenbeeinträchtigung mit großer Sicherheit vermieden. Bei Bemessung der Ausnehmung derart, daß sie in Umfangsrichtung einen Bogen entsprechend einem Winkel von weniger als 120° auf der zylindermantelförmigen Nageloberfläche umfaßt bzw. sie in Längsrichtung des Nagels auf eine entsprechende Länge begrenzt ist, kann der Nagel in eingetriebenem Zustand nahezu jede Lage einnehmen, ohne daß eine die Kortikalis gefährdende unzulässig hohe Flächenpressung entsteht.

Besonders vorteilhaft bei der erfindungsgemäßen Lösung ist neben der Tatsache, daß zur Herstellung der derart ausgebildeten Knochennägel nur unkomplizierte Werkzeuge benötigt werden weiterhin, daß auch ihre Manipulation mit einfach gestalteten Vorrichtungen möglich ist. So ist insbesondere eine zylinderförmig verrundete Ausnehmung unkompliziert durch Kaltverformung herstellbar und es läßt sich in einem Arbeitsgang auch eine im Bereich der Ausnehmung elliptisch oder ovale Ausbildung des Nagelquerschnitts erzielen, über die durch Aufstecken eines einfachen Werkzeugs ein Drehmoment übertragbar ist.

Bevorzugterweise ist weiterhin die Ausnehmung mit einer Durchtrittsöffnung versehen, die zu einem Bereich der Oberfläche des Nagels führt, die dem Ort der Ausnehmung gegenüberliegt, wobei die Durchtrittsöffnung einen Querschnitt aufweist, der wesentlich kleiner ist als der Oberflächenbereich des Nagels, der von der Ausnehmung eingenommen wird, so daß ein das Werkzeug beim Eingriff zur Manipulation des Nagels führender bzw. zentrierender Stift aufgenommen werden kann.

Ein Werkzeug zum Zurückziehen bzw. zum Ausüben eines Drehmoments auf einen erfindungsgemäßen Nagel weist bevorzugt einen Backen auf, der mit einer Erhebung versehen ist, welche an die Ausnehmung angepaßt ist, so daß eine Drehmoment- und Kraftübertragung erfolgen kann, ohne daß der Nagel von seiner runden Form abweichende, die Kortikalis gefährdende Vorsprünge aufweisen muß. Hierbei enthält der Backen eine Fläche, mittels der eine Rückzugskraft auf diejenige Fläche der Ausnehmung ausübbar ist, welche einen Teil einer Querschnittsfläche des Nagels bildet.

Günstigerweise ist an dem Werkzeug ein Sperrelement vorgesehen, welches sich an der der Ausnehmung gegenüberliegenden Teil der zylindermantelförmigen Oberfläche des distalen Nagelendes abstützt und die genannten Flächen in gegenseitigem Eingriff hält. Das Sperrelement wird dabei durch eine Nase, bzw. bei zangenartiger Ausbildung des Werkzeugs durch einen Gegenbacken und/oder durch einen verschiebbaren Riegel gebildet.

Durch die Erfindung wird insbesondere das Problem gelöst. daß Bündelnagelung die Knochennägel möglichst platzsparend angeordnet sein müssen, wobei die zur Aufbringung von Kräften auf den Nagel beim Einschlagen oder Herausziehen desselben notwendige Formelemente derart auszubilden, daß sie einen nur unwesentlichen zusätzlichen Raum einnehmen.

Weiterhin ist es bei dem erfindungsgemäßen Nagel vorteilhaft, daß die Position der Nägel im Knochen mittels einfacher in die Ausnehmung eingreifender an diese angepaßte Elemente fixierbar ist. Diese Ausnehmung ist bei den eingebrachten Nägeln von der Knochenaußenseite her zugänglich, so daß insoweit die Handhabung erleichtert ist.

Bei der Bündelnagelung wird bevorzugt eine « Plombierung » mit Knochenzement vorgenommen, wobei die noch nicht ausgehärtete Zementmasse mehrere Ausnehmungen von Nägeln mit benachbarter Knochensubstanz verbinden kann. Da sich der Knochenzement in die Ausnehmungen mit einem Querschnitt einfügt, der in Lage ist, die entsprechenden Schubspannungen aufzunehmen, werden somit Bewegungen der Nägel in Längsrichtung sicher verhindert.

Vorteilhafte Weiterbildungen der Erfindung sind in den Unteransprüchen gekennzeichnet bzw. werden bei der nachstehenden Darstellung einer bevorzugten Ausführung der Erfindung näher beschrieben. Es zeigen :

Figur 1 ein Ausführungsbeispiel des erfindungsgemäßen Knochennagels in Seitenansicht, Fig. 1a bis c das distale Ende eines Ausführungsbeispiels des erfindungsgemäßen Nagels in drei vergrößerten Seitenansichten,

Figur 2a dasselbe Ausführungsbeispiel im Querschitt,

Figur 2b eine Anordnung mehrerer der in Fig. 2a dargestellten Nägel im Bündel sowie,

Figur 3 ein Werkzeug zum Ausüben einer Kraft bzw. eines Drehmoments auf einen derartigen Nagel im Schnitt sowie.

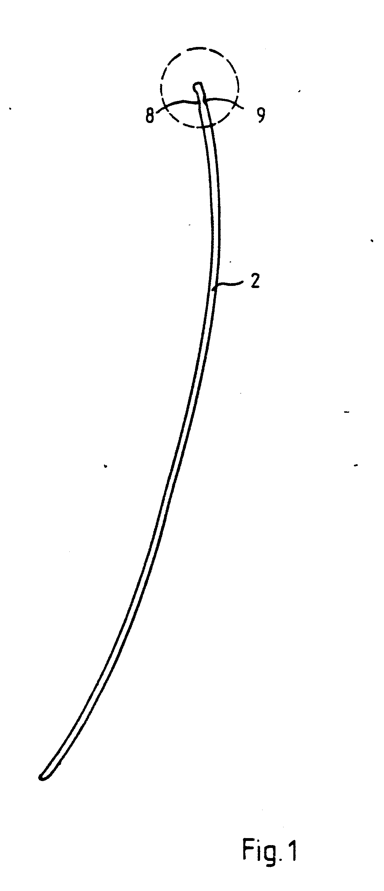

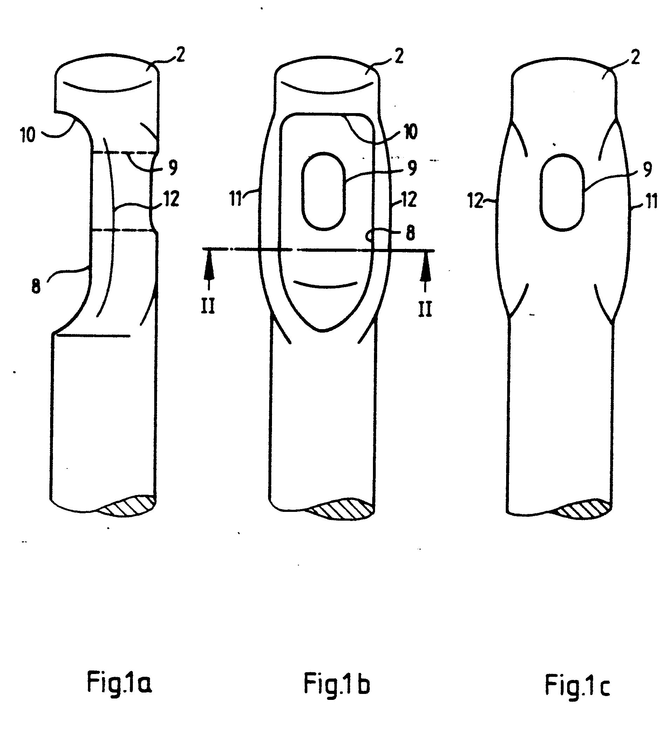

In Figur 1 ist ein Nagel 2 dargestellt, wobei der von den Figuren 1a bis 1c erfasste und vergrößert wiedergegebene Bereich von einer gestrichelten Linie umgeben ist. Eine Aussparung 8 des Nagels 2 ist an der Innenseite der Krümmung des Nagels angeordnet. Diese Krümmung kann sich aus verschiedenen Biegungen und Abbiegungen zusammensetzen, welche über die Gesamtlänge des Nagels verteilt sind, so daß bei der Anwendung zur Fixierung von Oberschenkelhalsfrakturen das die Aussparung 8 tragende Nagelende mit seiner der Aussparung gegenüberliegenden Rückseite schonend auf der Kortikalis anliegt.

Bei dem in den Figuren 1a bis c dargestellten distalen Ende des erfindungsgemäßen Nagels 2, das vergrößert wiedergegeben ist, ist eine halbrundförmige Ausnehmung 8, deren Verrundung in Richtung der Rundung der Nageloberfläche verläuft, — in Bezug auf dessen äußerstes Ende zurückversetzt — angeordnet. Die Ausnehmung 8 geht in ihren sich in Längsrichtung des Nagels erstreckenden Randbereichen in weichgeschwungener Formgebung in die Zylindermantelfläche des Nagels über. Auf dem Grunde der Ausnehmung ist eine Durchtrittsöffnung 9 vorgesehen, welche einen Durchlaß zum gegenüberliegenden Bereich der Mantelfläche des Nagels bildet und eine kleinere Fläche einnimmt als die Ausnehmung. Die Ausnehmung 8 weist eine obere Begrenzungsfläche 10 auf, die im wesentlichen horizontal verläuft. An diese gebildet ist und deren Fläche schließt direkt oder über eine Stufung der dem distalen Ende des Nagels nahegelegene Bereich der Durchtrittsöffnung 9 an. Zur Einleitung eines Drehmoments dienende Bereiche 11 und 12 sind seitlich der Ausnehmung 8 vorgesehen, welche vorzugsweise einseitig angebracht ist.

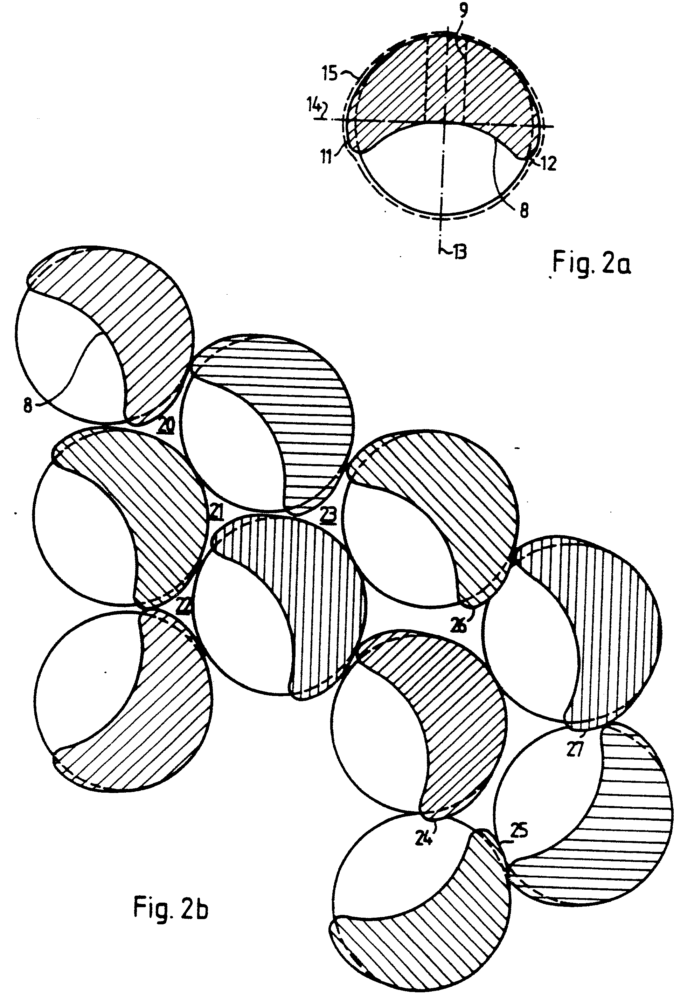

In Figur 2a ist der in den Figuren 1 bis 1c dargestellte Knochennagel im Querschnitt wiedergegeben. Die Schnittebene ist in Figur 1b mit II gekennzeichnet. Die Aussparung 8 ist dabei durch Kaltverformung in der Weise erzeugt worden, daß die Bereiche 11 und 12 sich über den kreisförmigen Nagelquerschnitt hinaus erstrecken und symmetrisch in Bezug auf eine strichpunktiert dargestellte, einen Durchmesser markierende Linie 13 angeordnet sind. Die Bereiche 11 und 12, die sich vorwölben und in Längsrichtung des Nagels über eine Strecke in Längsrichtung des Nagels ihren Querschnitt nicht verändern bzw. eine maximale Außenkontur nicht überschreiten, sind weiterhin in dem über den Kreisquerschnitt des Nagels hinausragenden Bereich symmetrisch zu einer Linie 14 ausgebildet, welche ebenfalls strichpunktiert dargestellt ist, so daß der Zugriff durch ein Werkzeug ermöglicht ist, welches in seiner inneren Kontur durch eine gestrichelte Linie 15 angedeutet ist. Das Werkzeug läßt sich auf diese Weise leicht aufstecken und handhaben, was im übrigen auch durch eine (um 120 bzw. 180°) radial symmetrisch ausgebildete Anordnung der Bereiche 11 und 12 erzielbar ist.

Aus dieser Schnittdarstellung ist ersichtlich, daß der Querschnitt des Nagels im Bereich der Ausnehmung 8 eine geringfügige Abweichung von der Kreisscheibenform aufweist, wenn man einmal von der Ausnehmung 8 selbst und der Durchtrittsöffnung 9 absieht. Die Querschnittsform ist bevorzugt geringfügig elliptisch bzw. oval oder dreieckig verrundet ausgebildet, wobei im Falle elliptischer Gestaltung das Achsenverhältnis bevorzugt im Bereich zwischen 1 zu 1,1 und 1 zu 1,2 gewählt ist und die längere Halbachse parallel zu demjenigen Oberflächenbereich gerichtet ist, an der die Ausnehmung 8 vorgesehen ist. Eine derartige geringfügige exzentrische Verformung des Querschnitts des Nagelendes im Bereich der Ausnehmung 8 läßt sich entsprechend einem bevorzugten Herstellungsverfahren für den erfindungsgemäßen Nagel durch Einschlagen der Ausnehmung im Wege der Kaltverformung erzielen. Die sich ergebende Exzentrizität ist ausreichend, um beim Einschlagen des Nagels mittels einer entsprechend angepaßten, auf diesen aufzusetzenden Hülse ein Drehmoment aufzubringen, welches ein gesteuertes Führen des Nagels bezüglich der an seinem proximalen Ende einzuschlagenden Richtung ermöglicht.

Es ist aus Figur 2a ferner ersichtlich, daß die Ausnehmung 8 derart angeordnet ist, daß sie aufgrund der erfindungsgemäßen Verrundungen in ihren seitlichen Bereichen keinerlei Kanten oder Flächen aufweist, welche einen meißelartigen Druck auf Knochen- oder Gewebeteile ausüben könnten. Eine derartige Einwirkung von eng begrenzten Flächenteilen mit dem daraus resultierenden hohen Anpreßdruck auf Knochen- oder Gewebebereiche ist auch dadurch verhindert, daß die Ausnehmung 8 nicht unmittelbar am Ende des Nagels, sondern um eine geringe Entfernung von dem Ende zurückversetzt angeordnet ist, so daß sich am unmittelbaren Nagelende ein im wesentlichen unverformter Bereich der Nageloberfläche befindet, der sich großflächig am Knochen bzw. Gewebe abstützen kann.

Die der Ausnehmung 8 gegenüberliegende Außenfläche des Nagels bildet einen Bereich, der auch bei axial um mehr als ± 90° verdrehter Einbringung des Nagels eine sichere Anlage am oberen Rand eines Knochenfensters gewährleistet. Wegen der relativ geringfügigen Abweichung des Nagelendes vom Kreisquerschnitt ist eine Parallelanordnung der Nägel in Bündellage auch im Bereich der Ausnehmung 8 ohne merkbaren zusätzlichen Raumbedarf möglich.

In Figur 2b ist eine Vielzahl von Nägeln 8 in willkürlicher bündelartiger Anordnung im Schnitt wiedergegeben, wobei an verschiedenen Berührungsbereichen benachbarter Nägel gezeigt werden soll, daß trotz der den Kreisquerschnitt der Nägel überschreitenden Bereiche 11 und 12 eine Nagelanordnung möglich ist, bei der der behandelnde Chirurg nicht darauf achten muß, ein genaue Ausrichtung der Nägel in bezug auf die Richtung der Ausnehmungen 8 zu erreichen. Vielmehr ist eine dichte Bündelpackung auch dann möglich, wenn die einzelnen Nägel um Winkelbeträge unterschiedlich angeordnet sind.

Dabei erstrecken sich an den Orten von sich zwischen benachbarten, dicht angordneten Nägeln befindlichen Hohlräume 21 bis 25 die den Kreisquerschnitt überschreitenden Bereiche 11 und 12 der Nägel 2. Sie ragen in die nicht von Material ausgefüllten Räume zwischen den Kreisquerschnitten der Nägel hinein, so daß die Nägel in dichtester Packung angeordnet sein können. Im Bereich 22 sind in dem durch das Aneinandergrenzen von drei Nägeln verbleibenden Aussparungsbereich die Wölbungsbereiche zweier Nägel angeordnet, welche sich ebenfalls nicht behindern.

Aus Figur 2b ist ersichtlich, daß das Anlehnen der distalen Nagelenden an der Kortikalis nicht entlang einer geraden Linie zu erfolgen braucht, sondern daß hier auch einer Krümmung ohne weiteres gefolgt werden kann. (Die Begrenzungslinie für den für die Nagelung zur Verfügung stehenden Raum kann dabei eine die in Figur 2b oben wiedergegebenen Nägel tangierende Linie gedacht werden.) Die dargestellten Vorteile der raumsparenden Anordnung bei dichter Bündelpackung beziehen sich auch auf die entsprechenden, im Bereich der entgegengesetzten Nagelenden (in den Zeichnungen nicht wiedergegebenen) Bereiche.

Weiterhin soll darauf hingewiesen werden, daß der zwischen den Kreisquerschnitten der Nägel zur Verfügung stehende Raum auch dann von den vorgewölbten Bereichen 11 und 12 eingenommen werden kann, wenn die Aussparungen 8 sich nicht in der selben Höhe befinden.

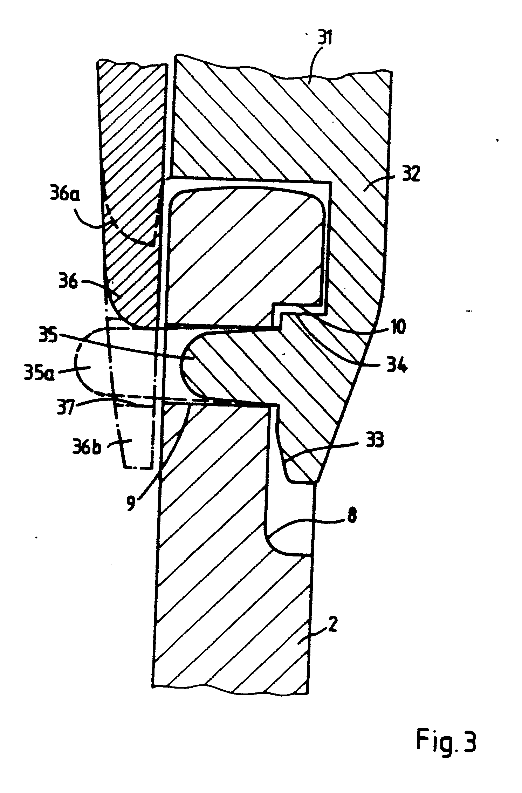

In Figur 3 ist der untere Bereich eines Werkzeugs 31 dargestellt, welches sich zur Ausübung von Druck- und Zugkräften bzw. Drehmomenten in Eingriff mit dem distalen Ende des Nagels 2 befindet. In der Figur sind mehrere Varianten gestrichelt wiedergegeben, welche in unterschiedlicher Kombination Anwendung finden können.

Ist das Ende des Nagels 2' im Bereich der Aussparung oval der elliptisch ausgeführt, so wird zweckmäßig als Schlageisen ein Instrument verwendet, welches (abweichend von in Figur 3 dargestellten Form) eine an die unrunde Form des Nagelendes angepaßte Aussparung enthält und somit beim Einschlagen die zur Drehung des Nagels erforderlichen Momente übertragen kann.

Andererseits lassen sich bei unrundem und auch bei kreisrundem Querschnitt sowohl zum drehenden Einschlagen als auch zum Zurückziehen des Nagels Werkzeuggestaltungen verwenden, wie sie in Figur 3 dargestellt sind.

An einem Trägerteil 32 des Werkzeugs 31 ist ein hakenförmiger Ansatz vorgesehen, welcher mit einem Backen 33 versehen ist, der an die Ausnehmung 8 angepaßt ist. Eine Fläche 34, welche auf die Fläche 10 des Nagels 2 einwirkt, ermöglicht dabei ein Zurückziehen des Nagels aus dem Knochen nach abgeschlossenem Heilerfolg.

Zur Führung des hakenförmigen Endes des Teils 32 des Werkzeugs 11 beim Ergreifen des distalen Endes des Nagels 2 dient ein Stift 35, der in verschiedener Länge (gestrichelte Linie 35a) vorgesehen sein kann und zur Erleichterung des Einführens in die Durchtrittsöffnung 9 an seinem freien Ende verrundet ausgebildet ist.

Um ein Abgleiten des hakenförmigen Ansatzes mit der Fläche 34 des Werkzeugs 31 beim Aufbringen eines Drehmoments oder beim Zurückziehen zu verhindern, ist an dem mit der Ausnehmung 8 in Kontakt kommenden Teil des Werkzeugs 31 eine Sperre 36 vorgesehen, welche verschiedenartig ausgebildet sein kann. Die als durchgezogene Linie dargestellte Variante kann in Form eines vom nicht dargestellten oberen Ende des Werkzeugs her betätigbaren und in Richtung des Nagels 2 verschiebbarer Riegel gestaltet oder aber den Gegenbacken einer Zange darstellen, mit der das distale Ende des Nagels 2 ergriffen werden kann.

Andererseits ist auch eine (gestrichelt dargestellte) Variante möglich, die fest mit der gegenüberliegenden, in die Ausnehmung 8 eingreifenden Seite des Werkzeugs 31 verbunden, aber verkürzt und geringfügig verrundet ausgebildet ist, so daß das einstückig feststehend gestaltete Werkzeug 31 mit seitlicher Neigung dem Nagelende « übergestülpt » werden kann. Wird das Werkzeug koaxial zum Nagel geführt, ist ein Abrutschen des in die Ausnehmung 8 eingreifenden Teil 33 sicher verhindert.

Bei zangenförmiger Ausgestaltung des Werkzeugs 31 ist weiterhin eine Variante günstig, welche als strichpunktierte Linie 36b wiedergegeben ist, wobei diese verlängerte Ausführung mit einem Durchbruch 37 versehen ist, welcher mit dem verlängerten Stift 35a in Eingriff kommt, so daß in Fällen, bei denen zum Herausziehen des Nagels besonders große Kräfte aufzubringen sind, die Festigkeit der Verbindung Werkzeug/Nagel zusätzlich erhöht ist.

### Patentansprüche

1. Elastischer Nagel (2) mit rundem Querschnitt zum Fixieren einer Oberschenkelfraktur und einem vom runden Querschnitt abweichenden Bereich in der Nähe eines seiner Enden zwecks Einleitung von Kräften und/oder Momenten beim Manipulieren — vorzugsweise zur Anwendung nach dem Prinzip der sogenannten « Bündelnagelung » mit einer Krümmung und Elastizität derart, daß sich der ins Markrohr eingetriebene Nagel mit seinem distalen Ende auf der Kortikalis abstützt — wobei der vom runden Querschnitt abweichende Bereich eine konkave und eine konvexe Fläche aufweist, die vorwiegend um parallel zur Längsrichtung des Nagels verlaufende Achsen gekrümmt sind, und den runden Querschnitt zum Teil überragt, dadurch gekennzeichnet, daß die konvexe Fläche die übrige zylindrische Außenoberfläche des Nagels lediglich geringfügig überragt und mit kontinuierlich gekrümmten Oberflächenbereichen an eine die konkave Fläche einschließende wannenförmige Ausnehmung (8) anschließt, wobei sich die Ausnehmung (8) bei gekrümmtem Nagel an der Innenseite der auf die Gesamtlänge des Nagels (2) bezogenen Krümmung befindet.

2. Nagel nach Anspruch 1, dadurch gekennzeichnet, daß den runden Querschnitt des Nagels überragenden Bereiche (11 und 12), welche als Angriffsflächen zur formschlüssigen Übertragung von Drehmomenten auf den Nagel dienen, nur so weit über die zylindermantelförmige Nageloberfläche hinausragen, daß mindestens ein derartiger Bereich in einem sich bei dichter Bündelpackung der Nägel zwischen ihren kreisförmigen Querschnitten verbleibende Raum (21 bis 25) Platz findet.

3. Nagel nach einem der vorangehenden Ansprüche, dadurch gekennzeichnet, daß die den runden Querschnitt überragenden Bereiche (11 und 12) in bezug auf Winkel von 120 bzw. 180° radial symmetrisch angeordnet sind.

4. Nagel nach einem der vorangehenden Ansprüche, dadurch gekennzeichnet, daß die Ausnehmung (8) und die den runden Querschnitt überragenden Bereiche (11 und 12) durch Kaltverformung hergestellt sind.

5. Nagel nach einem der vorangehenden Ansprüche, dadurch gekennzeichnet, daß diejenige Fläche (10) der Ausnehmung (8), welche eine Begrenzung zum nahen Nagelende darstellt, eben ist und einen Teil einer im wesentlichen rechtwinklig zur Achse gerichteten Querschnittsfläche des Nagels bildet.

6. Nagel nach einem der vorangehenden Ansprüche, dadurch gekennzeichnet, daß im Bereich der Ausnehmung (8) eine Durchtrittsöffnung (9) vorgesehen ist, die zu einem Bereich der Oberfläche des Nagels (8) führt, die der Ausnehmung gegenüberliegt, mit einem Querschnitt, der wesentlich kleiner ist als der Oberflächenbereich des Nagels (2), der von der Ausnehmung eingenommen wird.

7. Werkzeug (31) zum Ausüben einer Kraft oder eines Drehmoments auf den Nagel (2) nach einem der vorangehenden Ansprüche, dadurch gekennzeichnet, daß ein Backen (33) mit einer Erhebung vorgesehen ist, welche an die Ausnehmung (8) angepaßt ist.

8. Werkzeug nach Anspruch 7, dadurch gekennzeichnet, daß der Backen (33) eine Fläche (34) aufweist, mittels der eine Kraft auf diejenige Fläche (10) der Ausnehmung (8) ausübbar ist, welche einen Teil einer Querschnittsfläche des Nagels bildet.

9. Werkzeug nach Anspruch 8, dadurch gekennzeichnet, daß ein Sperrelement (36, 36a, 36b) vorgesehen ist, welches sich an einen der Ausnehmung (8) gegenüberliegenden Teil der zylindermantelförmigen Oberfläche des distalen Nagelendes abstützt und die Flächen (10 und 34) in gegenseitigem Eingriff hält.

10. Werkzeug nach Anspruch 9, dadurch gekennzeichnet, daß das Sperrelement aus einer Nase (36a), bei zangenartiger Ausführung des Werkzeugs aus einem Gegenbacken (36b) und-/oder aus einem verschiebbaren Sperriegel besteht.

11. Werkzeug nach einem der Ansprüche 7 bis 11 zur Verwendung mit einem Nagel nach Anspruch 6, dadurch gekennzeichnet, daß als Zentrierungselement ein Stift (35, 35a) vorgesehen ist, welcher bei in Eingriff befindlichem Backen in die am Nagel vorgesehene Durchtrittsöffnung eingreift bzw. durch diese hindurchgreift.

## Claims

1. A resilient nail (2) with a round cross-section for fixing a fracture of the femur having a region deviating from the round cross-section in the vicinity of one of its ends for the purpose of introducing forces and/or moments during manipulation — preferably for use in accordance with the principle of so-called « bunch nailing », with such a curvature and resilience that the nail driven into the narrow tube is supported on the cortex by its distal end — wherein the region deviating from the round cross-section comprises a concave and a convex surface which are predominantly curved about axes extending parallel to the longitudinal direction of the nail, and partially projects beyond the round cross-section, characterised in that the convex surface projects only slightly beyond the rest of the cylindrical surface of the nail and is joined, by continuously curved surface regions, to a trough-shaped recess (8) including the concave surface, and when the nail is curved, the recess (8) is at the inside of the curvature in relation to the total length of the nail (2).

2. A nail as claimed in Claim 1, characterised in that the regions (11 and 12) which project beyond the round cross-section of the nail and serve as working surfaces for the positive transmission of torque to the nail, only project so far beyond the cylindrical envelope of the nail that at least one such region finds room in a space (21 to 25) remaining between their circular corss-sections in a densely bunched pack of the nails.

3. A nail as claimed in one of the preceding Claims, characterised in that the regions (11 and 12) projecting beyond the round cross-section are disposed radially symmetrically in relation to angles of 120 or 180°.

4. A nail as claimed in one of the preceding Claims, characterised in that the recess (8) and the regions (11 and 12) projecting beyond the round cross-section are produced by cold-forming.

5. A nail as claimed in one of the preceding Claims, characterised in that that surface (10) of the recess (8) which represents a boundary at the near end of the nail is plane and forms part of a cross-sectional area of the nail directed substantially at right angles to the axis.

6. A nail as claimed in one of the preceding Claims, characterised in that in the region of the recess (8) there is a passage (9) which leads to a region of the surface of the nail (8) which is situated opposite to the recess and has a cross-section which is substantially smaller than the surface region of the nail (2) which is occupied by the recess.

7. A tool (31) for exerting a force or a torque on the nail (2) as claimed in one of the preceding Claims, characterised in that a jaw (33) is provided with a raised portion which is adapted to the recess (8).

8. A tool as claimed in Claim 7, characterised in that the jaw (33) comprises a surface (34) by means of which a force can be exerted on that surface (10) of the recess (8) which forms part of a cross-sectional area of the nail.

9. A tool as claimed in Claim 8, characterised in that a locking element (36, 36a, 36b) is provided which is supported on a portion of the cylindrical surface of the distal end of the nail situated opposite to the recess (8) and holds the surfaces (10 and 34) in mutual engagement.

10. A tool as claimed in Claim 9, characterised in that the locking element consists of a nose (36a), in the case of a pincer-like construction of the tool of a counter-jaw (36b) and/or of a displaceable locking bolt.

11. A tool as claimed in one of the Claims 7 to 11 for use with a nail as claimed in Claim 6, characterised in that a pin (35, 35a) is provided as a centring element and, when the jaws are in engagement, engages in the passage provided in the nail or engages through it.

**Revendications**

1. Clou élastique (2) présentant une section ronde, pour fixer une fracture du col du fémur et présentant, au voisinage de l'une de ses extrémités, une zone qui s'écarte de la section ronde dans le but de transmettre des forces et/ou des moments lorsqu'on le manipule — de préférence pour l'employer selon le principe de ce que l'on appelle l'enclouage en faisceau avec une cambrure et une élasticité telles que le clou introduit dans le canal médullaire s'appuie, par son extrémité distale, sur le tissu cortical étant précisé que la zone qui s'écarte de la section ronde présente une surface concave et une surface convexe qui sont principalement cambrées autour d'axes courant parallèlement à la direction longitudinale du clou et déborde partiellement de la section ronde, caractérisé en ce que la surface convexe ne déborde que légèrement au-delà de la surface extérieure, par ailleurs cylindrique, du clou et se raccorde, par des zones de surface cambrées en continu, à un évidement (8) en forme de cuvette contenant la surface concave, étant précisé que, lorsque le clou est cambré, l'évidement (8) se trouve sur la face intérieure de la cambrure rapportée sur toute la longueur du clou (2).

2. Clou selon la revendication 1, caractérisé en ce que les zones (11 et 12) qui débordent au-delà de la section ronde du clou et qui servent comme surfaces d'attaque pour transmettre, de par la forme, des moments de rotation au clou, ne dépassent à l'extérieur, au-delà de la surface latérale cylindrique du clou, que dans la mesure où au moins une zone de ce type peut trouver sa place dans un espace (21 à 25) qui subsiste entre les sections circulaires des clous lorsque ceux-ci constituent un faisceau dense.

3. Clou selon l'une des revendications précédentes, caractérisé en ce que les zones (11 et 12) qui débordent au-delà de la section ronde sont disposées en symétrie radiale par rapport à un angle de 120 à 180°.

4. Clou selon l'une des revendications précédentes, caractérisé en ce que l'évidement (8) et les zones (11 et 12) qui débordent au-delà de la section ronde sont obtenus par formage à froid.

5. Clou selon l'une des revendications précédentes, caractérisé en ce que la surface (10) de l'évidement (8) qui représente une limite dans la direction de l'extrémité, proche, du clou, est plane et forme une partie d'une surface de section du clou dirigée sensiblement perpendiculairement à l'axe.

6. Clou selon l'une des revendications précédentes, caractérisé en ce que dans la zone de l'évidement (8) est prévue une ouverture de passage (9) qui conduit à une zone de la surface du clou (8) qui est située en face de l'évidement et dont la section est sensiblement plus faible que celle de la zone de surface du clou (2) qu'occupe l'évidement.

7. Outil (31) pour exercer une force ou un moment de rotation sur le clou (2) selon l'une des revendications précédentes, caractérisé en ce qu'il est prévu un mors (33) avec une éminence adaptée à l'évidement (8).

8. Outil selon la revendication 7, caractérisé en ce que le mors (33) présente une surface (34) au moyen de laquelle on peut exercer une force sur la surface (10) de l'évidement (8) qui forme une partie de la surface de section du clou.

9. Outil selon la revendication 8, caractérisé en ce qu'il est prévu un élément de verrouillage (36, 36a, 36b) qui s'appuie contre une partie, opposée à l'évidement (8), de la surface latérale cylindrique de l'extrémité distale du clou et qui maintient les surfaces (10 et 34) en prise réciproque.

10. Outil selon la revendication 9, caractérisé en ce que l'élément de verrouillage est constitué d'un talon (36a), et, dans le cas d'une exécution de l'outil en forme de pince, d'un contre-mors (36b) et/ou d'un pène coulissant.

11. Outil selon l'une des revendications 7 à 11, pour emploi avec un clou selon la revendication 6, caractérisé en ce que comme élément de centrage est prévu un téton (35, 35a) qui, lorsque le mors se trouve en prise, pénètre dans l'ouverture de passage prévue sur le clou ou traverse cette ouverture.

8

9

2

Fig. 1

Fig.1a      Fig.1b      Fig.1c

Fig. 2a

Fig. 2b

Fig. 3